# EUROPÄISCHE PATENTSCHRIFT

(11) **EP 3 285 077 B1**
(45) Veröffentlichungstag und Bekanntmachung des Hinweises auf die Patenterteilung: **30.09.2020**
(21) Anmeldenummer: 17184588.6
(22) Anmeldetag: 02.08.2017
(51) Int. Cl.: G01K 7/34, H01B 7/32

(54) **VERFAHREN ZUR ÜBERWACHUNG EINER LEITUNG AUF VERÄNDERTE UMGEBUNGSBEDINGUNGEN SOWIE MESSANORDNUNG ZUR ÜBERWACHUNG EINER LEITUNG AUF VERÄNDERTE UMGEBUNGSBEDINGUNGEN**
METHOD FOR MONITORING A CONDUCTOR FOR CHANGES IN AMBIENT CONDITIONS AND MEASURING ASSEMBLY FOR MONITORING A CONDUCTOR FOR CHANGES IN AMBIENT CONDITIONS
PROCÉDÉ DE SURVEILLANCE DES CONDITIONS ENVIRONNEMENTALES MODIFIÉES D'UN CONDUCTEUR ET SYSTÈME DE MESURE PERMETTANT DE SURVEILLER DES CONDITIONS ENVIRONNEMENTALES MODIFIÉES D'UNE CONDUCTEUR

(30) Priorität: 15.08.2016 DE 102016215173
(43) Veröffentlichungstag der Anmeldung: 21.02.2018
(73) Patentinhaber: LEONI Kabel GmbH, 91154 Roth (DE)
(72) Erfinder: JANSSEN, Bernd, 26169 Friesoythe OT Neuscharrel (DE)
(74) Vertreter: FDST Patentanwälte

(56) Entgegenhaltungen:
- WO-A1-2009/115127
- WO-A1-2015/091552
- DE-A1-102007 008 263
- DE-T2- 69 417 360
- US-A- 4 868 506

## Beschreibung

Die Erfindung betrifft ein Verfahren sowie eine Messanordnung zur Überwachung einer Leitung auf veränderte Umgebungsbedingungen.

Bei der Leitung handelt es sich hierbei um eine elektrische Leitung mit zumindest einem Leiter, welcher von einer Isolierung umgeben ist. Der von Isolierung umgebene Leiter wird nachfolgend auch als Ader bezeichnet. Die Isolierung weist eine vorgegebene Dielektrizitätskonstante auf. Eine Signalübertragung innerhalb des elektrischen Leiters wird von der Dielektrizitätskonstanten beeinflusst.

Häufig sind mehrere Adern zu einer Leitung zusammengefasst. Bei vielen Anwendungen, beispielsweise im automotiven Bereich, unterliegen Leitungen dabei unterschiedlichsten Belastungen, welche häufig bezüglich ihrer Dauer und Stärke unbekannt sind. Auch prinzipiell vielfältige und wechselreiche Umgebungsbedingungen, insbesondere Wärmeeinflüsse, können oftmals nicht oder nicht hinreichend abgeschätzt werden, um einen Verschleiß einer Leitung vorhersagen zu können. Um eine bestimmte Mindestlebensdauer garantieren zu können, wird eine Leitung typischerweise überdimensioniert ausgelegt.

Relevanter Stand der Technik wird in WO2009/115127A1, DE69417360T2 sowie DE102007008263A1 offenbart.

Ausgehend hiervon liegt der Erfindung die Aufgabe zugrunde, eine einfache und kostengünstige Überwachung einer Leitung auf veränderte Umgebungsbedingungen zu ermöglichen.

Die Aufgabe wird erfindungsgemäß gelöst durch ein Verfahren mit den Merkmalen gemäß Anspruch 1 sowie durch eine Messanordnung mit den Merkmalen des Anspruchs 9. Vorteilhafte Ausgestaltungen und Weiterbildungen sind Gegenstand der Unteransprüche. Dabei gelten die Ausführungen im Zusammenhang mit dem Verfahren sinngemäß auch für die Messanordnung sowie umgekehrt.

Das erfindungsgemäße Verfahren dient zur Überwachung einer Leitung auf veränderte Umgebungsbedingungen. Die Leitung weist eine Messleitung mit einer vorbestimmten Länge auf, die einen von einer Isolierung mit bekannter Dielektrizitätskonstante umgebenen Messleiter aufweist. Die Leitung kann dabei neben der Messleitung weitere Leitungskomponenten, insbesondere weitere elektrische Leitungsadern oder sonstige elektrische Leitungen aufweisen. Bei einer bevorzugten Ausführungsvariante weist die Leitung jedoch nur die Messleitung auf.

Beim Verfahren wird weiterhin ein analoges Signal mit einer definierten Frequenz erzeugt. Dieses Signal wird anschließend in ein Referenzsignal und ein Laufsignal aufgeteilt, wobei das Laufsignal nachfolgend zu der Aufteilung in den Messleiter eingespeist wird. Das in den Messleiter eingekoppelte Signal durchläuft den Messleiter vorzugsweise zweifach, durchläuft also eine Hinstrecke sowie eine Rückstrecke. Das in der Rückstrecke propagierende Signal, nachfolgend als Rücklaufsignal bezeichnet, wird anschließend mit dem zuvor abgetrennten Referenzsignal zusammengeführt. Anhand einer Phasenverschiebung zwischen dem Referenzsignal und dem Rücklaufsignal wird schließlich ein Maß für die veränderte Umgebungsbedingung ermittelt. Das Rücklaufsignal wird vorliegend als Teil des Laufsignals angesehen, welches in der Rückstrecke propagiert.

Bei dieser Umgebungsbedingung oder auch Umgebungsparameter handelt es sich insbesondere um die Temperatur. Der Begriff Umgebungsbedingung, bzw. Umgebungsparameter ist dabei allgemein zu verstehen. Gemäß einer ersten Ausführungsvariante handelt es sich um eine Umgebungsbedingung außerhalb der Leitung. In diesem Fall wird daher mithilfe der Messleitung eine weitere Komponente allgemein überwacht. Alternativ hierzu handelt es sich bei der Bedingung um eine innere Umgebungsbedingung, beispielsweise eine Bedingung der Leitung selbst, beispielsweise die Temperatur der Leitung. Im letztgenannten Fall, bei dem es sich also um eine inhärente, innere Bedingung der Leitung handelt, die überwacht wird, dient das Überwachungsverfahren daher zur Überwachung der Leitung selbst. Anhand der ermittelten Werte für die veränderte inhärente Bedingung werden weiter vorzugsweise Prognosen für den Verschleiß der Leitung abgeleitet.

Das hier vorgeschlagene Verfahren beruht grundsätzlich auf der Überlegung, dass eine Laufzeit des in den Messleiter eingespeisten Signals insbesondere von der Dielektrizitätskonstanten abhängt. Diese wiederum variiert mit den Umgebungsbedingungen, insbesondere der Temperatur. Eine sich verändernde Umgebungstemperatur wirkt sich daher über die Dielektrizitätskonstante auf die zu erwartende Laufzeit des Signals aus. Da der Messleiter eine vorgegebene, definierte Länge aufweist, führt eine veränderte Dielektrizitätskonstante zu einer veränderten (elektrischen) Weglänge für das Signal.

Durch die Überlagerung des Rücklaufsignals mit dem Referenzsignal und die unterschiedlichen, temperaturabhängigen Laufzeiten kann aus der durch die verschiedenen Laufzeiten resultierenden Phasenverschiebung zwischen Referenzsignal und dem Rücklaufsignal ein Rückschluss letztendlich auf die veränderte (Umgebungs-)Bedingung, insbesondere auf die sich verändernde Temperatur, gezogen werden. Zweckdienlicherweise wird durch eine entsprechende Kalibrierung auf eine absolute aktuelle Temperatur rückgeschlossen.

Von besonderer Bedeutung für das vorliegende Verfahren ist die Verwendung eines analogen Signals. Hierunter wird allgemein ein stufenloses Signal mit einem durchgehenden unterbrechungsfreien Signalverlauf angesehen, im Unterschied zu einem digitalen Signal, welches typischerweise lediglich eine stufenförmige Abfolge von (zwei) diskrete Zustände (0,1) aufweist. Dies hat den Vorteil, dass dieses im Vergleich zu digitalen Messimpulsen eine deutlich geringere Frequenz aufweist. Dies führt dazu, dass die Signaldämpfung des Laufsignals vergleichsweise gering ist.

In bevorzugter Ausgestaltung werden das Referenzsignal sowie das Rücklaufsignal zu einem resultierenden Signal überlagert und aus der Amplitude des resultierten Signals wird dann das Maß für die sich verändernde Bedingung ermittelt. Bei dem resultierenden Signal handelt es sich daher um das Summen- oder Differenzsignal, gebildet aus Referenz- und Rücklaufsignal. Aus dem Summen- oder Differenzwert der Amplitude wird - bei bekannter Ausgangsamplitude des Referenzsignals sowie des Rücklaufsignals - auf die Phasenbeziehung zwischen beiden Signalen und damit wiederum auf die temperaturbedingt veränderte Dielektrizitätskonstante und damit letztendlich auf die veränderte Temperatur rückgeschossen.

Bei dem Signal handelt es sich allgemein um ein periodisches Signal mit einer vorgegebenen Frequenz, insbesondere um ein sich kontinuierlich veränderndes Signal mit stetig ansteigender bzw. abfallender Amplitude, speziell ein Sinus- oder Cosinus-Signal. Daneben besteht grundsätzlich auch die Möglichkeit, andere Signalgeometrien wie beispielsweise Dreiecksignale zu verwenden.

Vorzugsweise weist das Rücklaufsignal unter vorbestimmten Normalbedingungen, beispielsweise also bei einer normalen Umgebungstemperatur von beispielsweise 20°C eine zu erwartende Laufzeit auf. Die Frequenz des Signals und damit auch die Frequenz des in den Messleiter eingekoppelten Laufsignals ist dabei derart gewählt und abgestimmt, dass beim Zusammenfügungspunkt, an dem das Rücklaufsignal und das Referenzsignal zusammengeführt, speziell addiert werden, sich ein vorgegebener, definierter Phasenunterschied einstellt. Bevorzugt wird für derartige Normalbedingungen ein Phasenunterschied von 90° oder 270° eingestellt. Durch diese Maßnahme lassen sich Zustandsänderungen, insbesondere Temperaturänderungen zuverlässig detektieren. Speziell lassen sich beispielsweise sowohl Temperaturerhöhungen als auch Temperaturerniedrigungen zuverlässig detektieren und unterscheiden. Bei dem Zusammenführen der beiden Signale werden diese insbesondere addiert, d.h. ihre Amplituden werden vektoriell addiert. Bei 0° Phasenversatz ergibt sich ein Maximum und bei 180° Phasenversatz ein Minimum.

Um eine möglichst eindeutige Aussage über die Umgebungsbedingung zu ermöglichen, ist dabei weiterhin vorgesehen, dass die Frequenz derart gewählt ist, dass eine Phasenverschiebung zwischen dem Referenzsignal und dem Rücklaufsignal dem Betrag nach ≤180° ist, d.h. die für einen vorgegebenen maximalen Messbereich (Temperaturbereich) zu erwartende maximale Phasenverschiebung ist ≤180°. Vorzugsweise wird eine maximal zu erwartende Phasenverschiebung auf kleiner gleich +/- 45° eingestellt. Die maximale Phasenverschiebung wird also vorgegeben und derart gewählt, dass sie zu einer maximale zu erwartenden Laufzeitdifferenz korreliert. Die maximal zu erwartende Laufzeitdifferenz wird dabei durch die maximal zu erwartende Änderung der (Umgebungs-)Bedingung bestimmt. Es wird daher quasi ein maximaler Überwachungsbereich für den zu überwachenden Parameter (Umgebungsbedingung) vorgegeben. Unter Berücksichtigung dieses maximalen Überwachungsbereichs und der definierten Länge des Messleiters wird hieraus im Vorfeld zunächst die maximal zu erwartende Laufzeitdifferenz zwischen den Laufzeiten bei maximalem und minimalem Wert der Umgebungsbedingung ermittelt. Diese Laufzeitdifferenz ist also für das Rücklaufsignal innerhalb des vordefinierten maximalen Wertebereichs für die Variation des (Umgebungs-)Parameters zu erwarten. Aus der so ermittelten maximalen Laufzeitdifferenz und einer vorgegebenen maximalen Phasenverschiebung wird dann die Frequenz des Signals abgeleitet und eingestellt.

Zweckdienlicherweise liegt dabei die Frequenz im Bereich von mehreren 10KHz bis maximal mehreren 10MHz. Vorzugsweise liegt die Frequenz insgesamt im Bereich zwischen 0,5 und 60MHz und vorzugsweise bei 30MHz. Dabei nimmt die vordefinierte Frequenz mit zunehmender Leitungslänge ab. Diese Abhängigkeit der Frequenz von der Leitungslänge ist von besonderem Vorteil, da eine Signaldämpfung üblicherweise bei höheren Frequenzen zunimmt, was sich also bei längeren Messleitern unter Umständen negativ auswirken kann. Durch die reziproke Beziehung zwischen Leitungslänge des Messleiters und Frequenz eignet sich das vorliegende Verfahren mit den analogen Messsignalen daher insbesondere bei größeren Leitungslängen.

Die Leitungslängen des Messleiters liegen allgemein vorzugsweise im Bereich von einigen Metern bis einige 10m. Grundsätzlich ist die Länge des Messleiters jedoch nicht beschränkt. Die Länge des Messleiters kann auch mehrere 10m bis hin zu 100m oder einige 100m betragen.

Für die Auswertung des überlagerten, resultierenden Signals, welches durch Summen- oder Differenzbildung erhalten ist, ist weiterhin von besonderer Bedeutung, dass die Amplitude des Rücklaufsignals bekannt ist. Diese wird zweckdienlicherweise im Vorfeld beispielsweise rechnerisch oder auch durch Vergleichsmessungen ermittelt und entsprechend bei der Auswertung berücksichtigt.

Sofern aufgrund beispielsweise besonders großer Leitungslängen oder sonstiger Einflüsse eine nur geringe Amplitude des Rücklaufsignals zu erwarten ist, so wird gemäß einer bevorzugten Ausgestaltung das Rücklaufsignal verstärkt, beispielsweise derart, dass es zumindest etwa die gleiche Amplitude wie das Referenzsignal aufweist.

Bei dem Messleiter bzw. der Messleitung handelt es sich vorzugsweise allgemein um eine Stichleitung, also eine Leitung, die von dem Laufsignal zweimal, also auf einer Hinstrecke sowie auf einer Rückstrecke durchlaufen wird.

Gemäß einer ersten Ausführungsvariante handelt es sich bei dem Rücklaufsignal dabei zweckmäßigerweise um einen reflektierten Anteil des Laufsignals. In dieser Ausführungsvariante ist der Messleiter endseitig offen, also elektrisch nicht kontaktiert und beispielsweise nicht an einem vorgegebenen Potenzial angeschlossen. An einem derartigen offenen Ende erfolgt in an sich bekannter Weise eine Reflexion des Signals. Das Ende ist alternativ mit Masse kurzgeschlossen, was einer zusätzlichen Phasenverschiebung von 180° entspricht Bei diesen Ausführungsvarianten werden die reflektierten Anteile des Laufsignals als das Rücklaufsignal bezeichnet.

Speziell bei dieser Ausführungsvariante wird das Rücklaufsignal zweckdienlicherweise in einen von dem Messleiter getrennten Rückleiter eingekoppelt. Hierzu wird vorzugsweise ein sogenannter Richtkoppler verwendet. In einer einfachsten Ausführungsvariante ist der Rückleiter einfach neben dem Messleiter angeordnet und die Einkopplung des Rücklaufsignals erfolgt durch den Effekt des Nebensprechens. Speziell bei dieser Ausführungsvariante, bei dem also das Rücklaufsignal in einen vom Messleiter getrennten Rückleiter eingekoppelt wird, ist eine Verstärkung des in den Rückleiter eingekoppelten Rücklaufsignals vorgesehen, bevor das über den Rückleiter bereitgestellte Rücklaufsignal mit dem Referenzsignal zusammengeführt wird.

Gemäß einer alternativen, besonders bevorzugten Ausgestaltung weist die Messleitung zwei Adern oder Leiter auf, die miteinander verbunden, also kurzgeschlossen sind. Bei der Messleitung handelt es sich wiederum vorzugsweise um eine Stichleitung, wobei dann die beiden Adern endseitig miteinander verbunden sind. Die Hinstrecke der Messleitung ist dabei durch die eine Ader und die Rückstrecke der Messleitung durch die andere Ader gebildet. In diesem Fall ist das Rücklaufsignal daher nicht zwingend ein reflektiertes Signal, sondern unmittelbar das über die Rückstrecke, also über die zweite Ader propagierende Laufsignal.

Die Messleitung weist dabei zweckdienlicherweise einen dritten Leiter auf, welcher insbesondere durch eine Schirmung gebildet ist und insbesondere mit einem Bezugspotenzial, beispielsweise Masse verbunden ist. Hierdurch ist quasi einen Rückpfad für das Wechselspannungs-Signal gebildet. In diesem Fall handelt es sich bei der Messleitung daher insgesamt vorzugsweise um eine zweiadrige geschirmte Leitung, bei der die zwei Adern endseitig miteinander kurzgeschlossen sind.

Gemäß einer dritten und einfachsten Variante ist schließlich die Messleitung einseitig am Sender und mit der anderen Seite direkt am Empfänger angeschlossen. Hierzu wird beispielswese eine Zweidrahtleitung oder auch eine Koaxleitung an einer Einspeiseseite sowie an einer Empfängerseite angeschlossen. Auch hier ist das Rücklaufsignal insbesondere das sich im Messleiter 24 ausbreitende Laufsignal LS, welches dann mit dem Referenzsignal RS verglichen wird.

Die Aufgabe wird erfindungsgemäß weiterhin gelöst durch eine Messanordnung, die insbesondere zur Durchführung des zuvor beschriebenen Verfahrens ausgebildet ist. Diese Messanordnung weist dabei folgende Komponenten auf:
- Eine Messleitung mit einer vorbestimmten Länge, die einen Messleiter aufweist, welcher von einer Isolierung umgeben ist, die eine temperaturabhängige Dielektrizitätskonstante aufweist,
- einen Signalgenerator zur Erzeugung eines analogen Signals mit vorgegebener Frequenz,
- einen Splitter zur Erzeugung eines Referenzsignals sowie aus dem Laufsignal bzw. aus dem analogen Signal,
- wobei der Messleiter an einem Ausgang des Splitters angeschlossen ist zur Einkopplung des Laufsignals in den Messleiter,
- eine Auswerteeinheit zur Ermittlung eines Maßes für die veränderte Bedingung auf Grundlage eines Phasenversatzes zwischen dem Referenzsignal und dem Rücklaufsignal.

Die Auswerteeinheit umfasst dabei zweckdienlicherweise ein Summier- oder Differenzglied, welches zur Überlagerung des Rücklaufsignals mit dem Referenzsignal ausgebildet ist. Insbesondere handelt es sich hierbei beispielsweise um einen Differenzverstärker. Das hieraus erhaltene resultierende Signal wird dann vorzugsweise einem Spannungsmesser zugeführt, mittels dem die Amplitude des resultierenden Signals ermittelt wird.

Vorzugsweise sind dabei sämtliche Einspeise- sowie Auswertekomponenten der Messanordnung innerhalb einer gemeinsamen Überwachungseinheit integriert. An dieser Überwachungseinheit, welche also eine Baueinheit ist und hierzu beispielsweise in einem gemeinsamen Gehäuse angeordnet ist oder auf einem gemeinsamen Träger, wie beispielsweise eine Platine angeordnet ist, ist lediglich die Messleitung angeschlossen. Speziell erfolgt dies über eine reversible Anschlussmöglichkeit an Kontaktanschlüssen.

Die Messleitung selbst ist dabei weiterhin in einer zu überwachenden Komponente verlegt, und zwar insbesondere zur Überwachung der Temperatur innerhalb der Komponente.

Bei der Komponente handelt es sich dabei gemäß einer ersten Ausgestaltung um ein Kabel, welches selbst überwacht werden soll. Die Messleitung ist daher innerhalb eines elektrischen Kabels integriert. Neben einer Temperaturüberwachung lässt sich das Kabel allgemein auch auf andere Bedingungen, insbesondere mechanische Belastungen überprüfen, insbesondere dann, wenn diese zu einem Bruch des Messleiters geführt haben.

Bei dem Kabel handelt es sich dabei insbesondere um ein Kabel, welches im Anwendungsfall in einem Kraftfahrzeug, beispielsweise im Motorbereich etc. verlegt ist. Ein derartiges Kabel weist typischerweise eine Länge von lediglich einigen Metern bis maximal etwa 10m auf.

Gemäß einer zweiten Ausführungsvariante handelt es sich bei der zu überwachenden Komponente um ein Bauteil, wie beispielsweise ein Motorenbauteil oder ein sonstiges Bauteil, dessen Temperatur überwacht und/oder kontrolliert werden soll.

Gemäß einer besonders bevorzugten Ausgestaltung handelt es sich bei der Komponente um eine Masse, wobei die Masse sich durch eine exotherme oder endotherme Reaktion verändert. Über die Messanordnung besteht daher die vorteilhafte Möglichkeit, die exo- oder endotherme Reaktion zu überwachen. Speziell handelt es sich bei der Masse um eine aushärtbare oder trockenbare Masse, speziell beispielsweise um eine Vergussmasse, insbesondere im Baugewerbe. Speziell handelt es sich bei dieser Masse um Beton. Anhand der Temperatur kann auf den Trocknungs- oder Aushärtegrad des Betons auch im Inneren zuverlässig geschlossen werden. Die Messleitung ist daher in der Masse eingebettet und verbleibt in dieser nach der Aushärtung der Masse.

Ausführungsbeispiele der Erfindung werden nachfolgend anhand der Figuren näher erläutert. Diese zeigen jeweils in vereinfachten Darstellungen:
- Fig. 1: eine erste Ausführungsvariante einer Messanordnung,
- Fig. 2: eine zweite Ausführungsvariante der Messanordnung sowie
- Fig. 3: eine dritte Ausführungsvariante der Messanordnung..

In den Figuren sind gleich wirkende Teile jeweils mit den gleichen Bezugszeichen versehen.

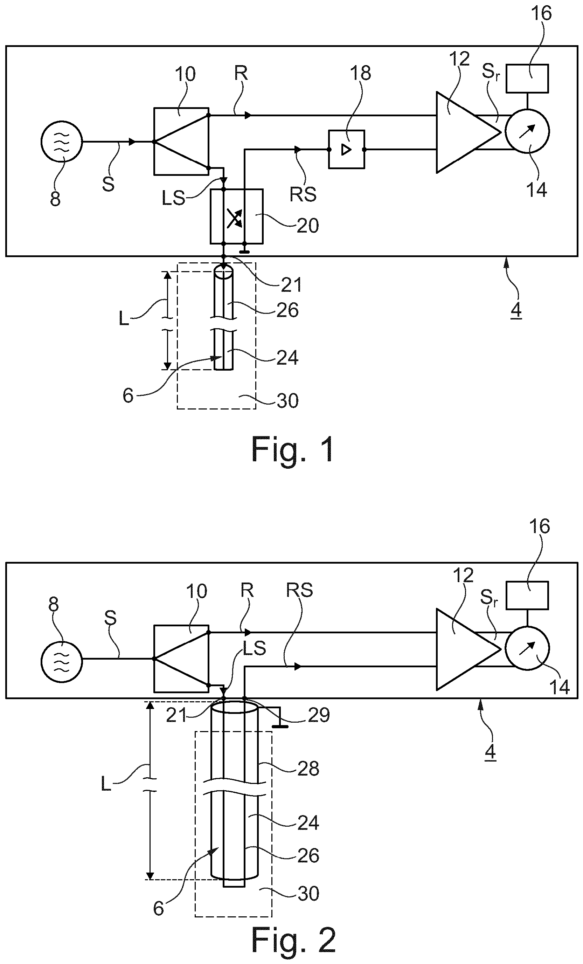

Gemäß Fig. 1 umfasst eine Messanordnung 2 eine Überwachungseinheit 4, an der eine Messleitung 6 angeschlossen ist. Die Überwachungseinheit 4 bildet daher eine bauliche Einheit. Innerhalb dieser Überwachungseinheit 4 ist ein Signalgenerator 8, ein Splitter 10, ein insbesondere als Differenzverstärker ausgebildeter Komparator 12, ein Spannungsmesser 14 sowie eine Auswerteeinheit 16 integriert. Ergänzend ist fakultativ noch ein Verstärker 18 sowie ein Koppelelement 20 integriert, welches vorzugsweise als Richtkoppler ausgebildet ist.

An diese Überwachungseinheit 4 ist die insbesondere als Stichleitung ausgebildete Messleitung 6 angeschlossen, beispielsweise an einen geeigneten Anschluss 21, wie z.B. Steckkontakt.

Bei der Messleitung 6 handelt es sich im Ausführungsbeispiel der Fig. 1 um eine einadrige Leitung mit einer Ader, also mit einem einen von einer Isolierung 22 umgebenen Messleiter 24. Durch die Ausgestaltung als Stichleitung ist das eine Ende der Messleitung 6 offen, der Messleiter 24 ist also nicht elektrisch kontaktiert.

Vom Signalgenerator 8 wird ein periodisches Wechselspannungssignal S, insbesondere ein Sinussignal, erzeugt. Dieses wird im Splitter 10 aufgeteilt auf ein Referenzsignal R sowie auf ein Laufsignal LS. Das Laufsignal LS wird nachfolgend in den Messleiter 24 eingespeist. Im Ausführungsbeispiel ist innerhalb der Überwachungseinheit 4 noch eine interne Leiterstrecke ausgebildet, welche vom Splitter 10 über das Koppelelement 20 zu dem Anschluss geführt ist, an dem der Messleiter24 angeschlossen ist.

Das Laufsignal LS wird wegen der Ausgestaltung als Stichleitung endseitig an dem offenen Ende der Messleitung 6 reflektiert. Der reflektierte Anteil propagiert als Rücklaufsignal RS innerhalb des Messleiters 24 in entgegengesetzter Richtung zum Laufsignal LS. Über das Koppelelement 20 wird das Rücklaufsignal RS innerhalb der Überwachungseinheit 4 in einen Rückleiter 26 eingekoppelt. Das darin eingekoppelte Rücklaufsignal RS wird über den fakultativen Verstärker 18 verstärkt und einem ersten Eingang des Komparators 12 zugeführt. An einem zweiten Eingang des Komparators 12 liegt das Referenzsignal R an. Der Komparator 12 erzeugt durch Summen- oder Differenzbildung ein resultierendes Signal S. Dessen Amplitude wird über den Spannungsmesser 14 erfasst und als Spannungsmesssignal der Auswerteeinheit 16 zugeführt.

Bei der Ausführungsvariante gemäß der Fig. 2 ist die Messleitung 6 durch eine zweiadrige und insbesondere mit einer Schirmung 28 versehene Leitung ersetzt. Die Leiter der beiden Adern sind dabei endseitig miteinander verbunden. Die Leiter der beiden Adern bilden gemeinsam den Messleiter 24.

Die eine Ader ist dabei an dem Anschluss 21 der Überwachungseinheit 4 angeschlossen. In diese Ader wird also das Laufsignal LS vom Splitter 10 eingespeist. Die andere Ader ist an einem weiteren als Eingang ausgebildeten Anschluss 29 der Überwachungseinheit 4 angeschlossen und wird innerhalb der Überwachungseinheit 4 über eine Leiterstrecke mit dem ersten Eingang des Komparators 12 verbunden. Das Referenzsignal R liegt wiederum am zweiten Eingang des Komparators 12 an. Wie auch bei der Ausführungsvariante gemäß der Fig. 1 wird mithilfe des Spannungsmessers 4 die Spannung (Amplitude) des resultierenden Signals Sr gemessen und einer Auswerteeinheit 16 zur Verfügung gestellt. Bei der Ausführungsvariante der Fig. 2 ist im Ausführungsbeispiel auf einen Verstärker 18 verzichtet.

Bei dieser Ausführungsvariante ist das am ersten Eingang des Komparators 12 anliegende Rücklaufsignal RS identisch zu dem Laufsignal LS, d.h. das Laufsignal LS wird über die beiden kurzgeschlossenen Leiter der Messleitung 6 quasi durch die Messleitung 6 geschleift und dem ersten Eingang des Komparators 12 als das Rücklaufsignal RS zugeführt.

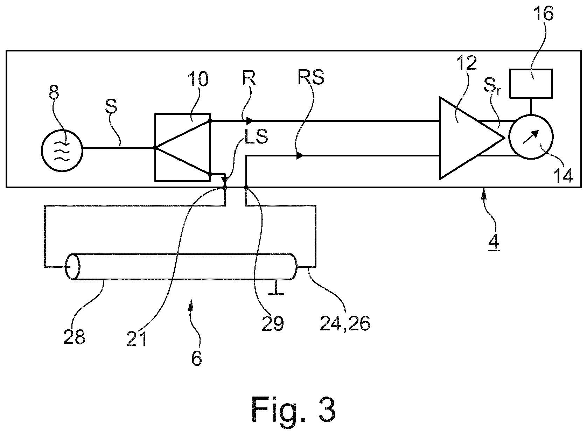

Fig. 3 zeigt schließlich eine weitere, besonders einfache Ausführungsvariante, bei der ein Leiter der Messleitung 6 als Messleiter 24 einerseits mit einer Einspeiseseite auf Seite des Signalgenerators 8 und andererseits mit einer Empfängerseite auf Seite der Auswerteeinheit 16 verbunden ist. Im Ausführungsbeispiel ist der Messleiter 24 an den beiden Anschlüssen 21,29 angeschlossen. Bei der Messleitung 6 handelt es sich beispielsweise um eine Koaxialleitung oder auch um eine Zweidrahtleitung. Bei der Koaxialleitung ist einer der Leiter (Innenleiter, Außenleiter) der Messleiter und der andere ist bevorzugt mit Bezugspotenzial (Masse) verbunden. Bei der Zweidrahtleitung ist ebenfalls einer der Leiter der Messleiter 24 und der andere bevorzugt mit Bezugspotenzial verbunden.

Die Messanordnung 2 dient insbesondere zur. Überwachung der Messleitung 6 auf eine veränderte Bedingung. Dies kann eine externe Umgebungsbedingung oder auch eine interne Bedingung der Messleitung 6 selbst sein. Hierzu wird mit Hilfe der Messanordnung insbesondere Zustandsparameter, speziell ein Umgebungsparameter überwacht. Bei diesem Zustandsparameter handelt es sich insbesondere um die Temperatur.

Das Messprinzip beruht dabei darauf, dass die Ausbreitung des eingekoppelten Messsignals innerhalb der Messleitung 6 und damit eine Laufzeit des Rücklaufsignals RS unter anderem auch von der Dielektrizitätskonstanten der Isolierung 22 abhängt. Die Dielektrizitätskonstante ist wiederum temperaturabhängig, sodass die Laufzeit des Rücklaufsignals RS temperaturabhängig ist. Unter Laufzeit des Rücklaufsignals RS wird dabei die gesamte Zeit verstanden, die das Signal (Laufsignal LS plus Rücklaufsignal RS) für die Strecke zwischen Ausgang des Splitters 10 bis zum ersten Eingang des Komparators 12 benötigt.

Untersuchungen haben gezeigt, dass beispielsweise bei einer Umgebungstemperatur von minus 40°C die Signalgeschwindigkeit des sich ausbreitenden Signals bei etwa 6ns pro Meter und bei einer Umgebungstemperatur von 105°C bei 8ns pro Meter liegt.

Die Messleitung 6 weist insgesamt eine definierte, vorgegebene Länge L auf. Diese Länge wird dabei in beiden Ausführungsvarianten zweimal durchlaufen. Beträgt die Länge L beispielsweise 10m, so beträgt die Laufstrecke für das Signal 20m.

Geht man von einem maximal zu überwachenden Wertebereich für den zu überwachenden Zustandsparameter aus, vorliegend beispielsweise von maximal etwa 145° für die zu überwachende Temperatur (-40°C bis 105°C), so ist insgesamt bei einer Leitungslänge L von 10m mit einem Laufzeitunterschied zwischen der minimalen Temperatur und der maximalen Temperatur von beispielsweise 40ns auszugehen. Dies entspricht einer zu erwartenden maximalen Laufzeitdifferenz Δt.

Zweckdienlicherweise unterscheiden sich die Signalwege für das Referenzsignal R und das Messsignal (Laufsignal LS plus Rücklaufsignal RS) durch den Signalweg durch die Messleitung 6. Die Signalwege innerhalb der Überwachungseinheit für das Referenzsignal R einerseits sowie für das Messsignal andererseits sind daher vorzugsweise identisch. Insgesamt sind die Signalwege für das Referenzsignal R sowie das Messsignal derart gewählt, dass sich beim Zusammenführen der beiden Signale bei definierten Normalbedingungen (z.B. bei 20° Celsius) bei einer "mittleren" Laufzeit von vorliegend beispielsweise 7ns eine definierte, vorgegebene Phasenverschiebung von beispielsweise 90° oder auch 270° einstellt.

Allgemein führt der durch die Messleitung 6 verlängerte elektrische Signalweg zu einer definierten Verschiebung der Phasenlage des Rücklaufsignals RS im Vergleich zu dem Referenzsignal R. Die Amplitude des resultierenden Signals Sr ändert sich daher in Abhängigkeit der Phasenlage der beiden Signale R, RS zueinander. Da diese wiederum von der Temperatur abhängt, lässt sich aus einer Phasenveränderung unmittelbar auch die Temperatur rückschließen.

Speziell bei einem stetigen Signal, insbesondere Sinussignal, überlagern sich die beiden Signalanteile an den Eingängen des Komparators linear. Insgesamt resultiert eine Spannungsveränderung des resultierenden Signals Sr, die zumindest im Wesentlichen proportional zu einer Temperaturänderung ist.

Um eine eindeutige Auswertung zu ermöglichen wird weiterhin die Frequenz des erzeugten Signals S derart gewählt, dass bei der zu erwartenden maximalen Laufzeitdifferenz Δt lediglich ein vorgegebener maximaler Phasenversatz zwischen den beiden Signalen auftritt. Dieser maximale Phasenversatz ist vorzugsweise ≤ +/- 90° und vorzugsweise ≤ +/- 45°.

Im obigen Beispiel, bei dem eine maximale Laufzeitdifferenz Δt von 40ns zu erwarten ist und bei einer gewünschten maximalen Phasenverschiebung von 90° (+/- 45°) ergibt sich also eine Periodendauer von 160ns für eine 360°-Periode. Daraus ergibt sich im Ausführungsbeispiel eine Frequenz für das Signal von etwa 6,25MHz.

Wird die Leitungslänge L um den Faktor 10 verändert, so ändert sich auch die Frequenz des einzuspeisenden Signals S um den Faktor 10, wobei mit zunehmender Länge L der Messleitung 6 die Frequenz verringert wird.

Die Messleitung 6 wird dabei innerhalb einer zu überwachenden Komponente 30 geführt. Bei dieser handelt es sich beispielsweise um ein Kabel, sodass also die Temperaturbelastung des Kabels gemessen und überwacht wird. In Abhängigkeit dieser Temperaturüberwachung wird dann zweckdienlicherweise auf eine zu erwartende Lebensdauer bzw. auf den Verschleiß des Kabels rückgeschlossen.

Bei einer zweiten Ausführungsform handelt es sich bei der Komponente 30 um eine zu überwachende Baueinheit.

Gemäß einer dritten Ausführungsvariante ist die Messleitung 6 innerhalb einer Masse eingebettet. Bei der Masse handelt es sich beispielsweise um Beton, d.h. die Messleitung 6 wird mit einbetoniert. Über die ganze Messanordnung 2 kann dann eine Temperatur beim Abbinden des Betons überwacht werden.

## Patentansprüche

1. Verfahren zur Überwachung einer Leitung auf veränderte Umgebungsbedingungen, wobei die Leitung eine Messleitung mit einer vorbestimmten Länge aufweist, die einen von einer Isolierung umgebenen Messleiter aufweist, wobei
- ein analoges Signal mit vorgegebener Frequenz erzeugt wird,
**dadurch gekennzeichnet, dass**
- das Signal in ein Referenzsignal und ein Laufsignal aufgeteilt wird wobei sich die Signalwege für das Referenzsignal und das Messsignal unterscheiden, wobei der elektrische Signalweg durch den Messleiter verlängert ist,
- das Laufsignal in den Messleiter eingespeist wird,
- das in den Messleiter eingespeiste Signal durchläuft eine Hinstrecke sowie eine Rückstrecke und ein aus dem Laufsignal erhaltenes, in der Rückstrecke propagierendes Rücklaufsignal wird mit dem Referenzsignal zusammengeführt , wobei
- eine Laufzeit des in den Messleiter eingespeisten Laufsignals von der vorbestimmten Länge des Messleiteres sowie von einer Dielektrizitätskonstanten der Isolierung des Messleiters abhängt, welche mit den Umgebungsbedingungen variiert, wobei eine veränderte Dielektrizitätskonstante zu einer veränderten elektrischen Weglänge führt
- anhand einer durch unterschiedliche Laufzeiten aufgrund der veränderten elektrischen Weglänge resultierende Phasenverschiebung zwischen dem Referenzsignal und dem Rücklaufsignal wird ein Maß für die veränderte Bedingung ermittelt.

2. Verfahren nach Anspruch 1, bei dem das Referenzsignal und das Rücklaufsignal zu einem resultierenden Signal überlagert werden und aus der Amplitude des resultierenden Signals das Maß für die veränderte Bedingung ermittelt wird.

3. Verfahren nach einem der Ansprüche 1 bis 2, bei dem es sich bei dem Signal um ein periodisches Signal mit vorgegebener Frequenz, insbesondere ein Sinussignal handelt.

4. Verfahren nach Anspruch 3, wobei das Rücklaufsignal unter Normalbedingungen eine zu erwartende Laufzeit aufweist und die Frequenz derart abgestimmt ist, dass sich bei Normalbedingungen ein definierter Phasenunterschied zwischen dem Rücklaufsignal und dem Referenzsignal einstellt, der vorzugsweise 90° oder 270° beträgt.

5. Verfahren nach einem der Ansprüche 3 oder 4, wobei das Rücklaufsignal unter Normalbedingungen eine zu erwartende Laufzeit und bei von den Normalbedingungen abweichenden Umgebungsbedingungen eine zu erwartende maximale Laufzeitdifferenz aufweist und wobei die Frequenz derart auf die zu erwartende maximale Laufzeitdifferenz abgestimmt ist, dass die Phasenverschiebung dem Betrage nach ≤180° ist, vorzugsweise ≤90° und insbesondere ≤45° ist.

6. Verfahren nach einem der Ansprüche 1 bis 5, wobei das Rücklaufsignal verstärkt wird, insbesondere derart, dass es die gleiche Amplitude wie das Referenzsignal aufweist.

7. Verfahren nach einem der Ansprüche 1 bis 6, wobei es sich bei dem Rücklaufsignal um einen reflektierten Anteil des Laufsignals handelt und die Messleitung als eine Stichleitung ausgebildet ist.

8. Verfahren nach einem der Ansprüche 1 bis 7, bei dem das Rücklaufsignal in einen vom Messleiter getrennten Rückleiter eingekoppelt wird.

9. Messanordnung zur Überwachung einer Leitung auf veränderte Umgebungsbedingungen, aufweisend
- eine Messleitung mit einer vorbestimmten Länge, die einen von einer Isolierung umgebenen Messleiter hat,
- einen Signalgenerator zur Erzeugung eines analogen Signals mit vorgegebener Frequenz,
**dadurch gekennzeichnet, dass** die Messanordnung weiter aufweist
- einen Splitter zur Erzeugung eines Referenzsignals sowie eines Laufsignals aus dem analogen Signal,
- wobei der Messleiter an einem Ausgang des Splitters angeschlossen ist zur Einkopplung des Laufsignals in den Messleiter,
- wobei sich die Signalwege für das Referenzsignal und das Messsignal unterscheiden und der elektrische Signalweg durch den Messleiter verlängert ist,
- eine Laufzeit des in den Messleiter eingespeisten Laufsignals von der vorbestimmten Länge des Messleiteres sowie von einer Dielektrizitätskonstanten der Isolierung des Messleiters abhängt, welche mit den Umgebungsbedingungen variiert, wobei eine veränderte Dielektrizitätskonstante zu einer veränderten elektrischen Weglänge führt,
- eine Auswerteeinheit zur Ermittlung eines Maßes für die veränderte Bedingung auf Grundlage eines durch unterschiedliche Laufzeiten aufgrund der veränderten elektrischen Weglänge resultierenden Phasenversatzes zwischen dem Referenzsignal und dem aus dem Laufsignal erhaltenen Rücklaufsignal.

10. Messanordnung nach Anspruch 9, die ein Summier- oder Differenzglied zur Überlagerung des Rücklaufsignals mit dem Referenzsignal aufweist.

11. Messanordnung nach Anspruch 9 oder 10, bei dem es sich bei der Messleitung um eine Stichleitung handelt.

12. Messanordnung nach einem der Ansprüche 9 bis 11 bei dem die Messleitung zwei miteinander endseitig kurzgeschlossene Adern aufweist und insbesondere als zweiadrige, mit einer Schirmung versehene Leitung ausgebildet ist.

13. Messanordnung nach einem der Ansprüche 9 bis 12, die eine Überwachungseinheit aufweist, an der die Messleitung über Kontaktanschlüsse angeschlossen ist, wobei in der Überwachungseinheit der Signalgenerator, der Splitter, der Empfänger sowie die Auswerteeinheit gemeinsam integriert sind.

14. Messanordnung nach einem der Ansprüche 9 bis 13, bei der die Messleitung in einer zu überwachenden Komponente verlegt ist, insbesondere zur Überwachung einer in der Komponente herrschenden Temperatur.

15. Messanordnung nach Anspruch 14, bei dem es sich bei der Komponente um ein Kabel, ein thermisch belastetes Bauteil oder um eine Masse handelt, welche sich durch eine exotherme- oder endotherme Reaktion verändert.

## Claims

1. Method for monitoring a line for changed ambient conditions, wherein the line comprises a measuring line with a predetermined length, which comprises a measuring conductor surrounded by an insulation, wherein
. an analog signal with a predetermined frequency is generated,
**characterized in that**
the signal is divided into a reference signal and a run signal, wherein the signal paths for the reference signal and the measuring signal differ, wherein the electrical signal path is extended by the measuring conductor,
- the run signal is fed into the measuring conductor,
- the signal fed into the measuring conductor passes through a forward path and a return path, and a return signal obtained from the run signal and propagating in the return path is combined with the reference signal, wherein
- a transit time of the run signal fed into the measuring conductor depends on the predetermined length of the measuring conductor and on a dielectric constant of the insulation of the measuring conductor, which varies with the ambient conditions, wherein a modified dielectric constant results in a modified electrical path length
- on the basis of a phase shift between the reference signal and the return signal resulting from different propagation times due to the changed electrical path length, a measure for the changed condition is determined.

2. Method according to claim 1, in which the reference signal and the return signal are superimposed to form a resulting signal and the measure of the changed condition is determined from the amplitude of the resulting signal.

3. Method according to one of claims 1 to 2, in which the signal is a periodic signal with a predetermined frequency, in particular a sinusoidal signal.

4. Method according to claim 3, wherein the return signal under normal conditions has an expected duration and the frequency is tuned in such way that under normal conditions a defined phase difference between the return signal and the reference signal is set, which is preferably 90 ° or 270 °.

5. Method according to one of claims 3 or 4, wherein under normal conditions the return signal has an expected transit time and under ambient conditions deviating from the normal conditions an expected maximum transit time difference, and wherein the frequency is tuned to the expected maximum transit time difference in such way that the phase shift in terms of magnitude is ≤180°, preferably ≤90° and in particular ≤45°.

6. Method according to one of claims 1 to 5, wherein the return signal is amplified, in particular in such way that it has the same amplitude as the reference signal.

7. Method according to one of claims 1 to 6, wherein the return signal is a reflected part of the run signal and the measuring line is designed as a stub line.

8. Method according to one of claims 1 to 7, in which the return signal is coupled into a return conductor separate from the measuring conductor.

9. Measuring arrangement for monitoring a line for changed ambient conditions, comprising
- a measuring line with predetermined length, having a measuring conductor surrounded by an insulation,
- a signal generator for generating an analog signal with a predetermined frequency,
**characterized in**
**that** the measuring arrangement further comprises
- a splitter for generating a reference signal and a run signal from the analog signal,
- wherein the measuring conductor is connected to an output of the splitter for coupling the run signal into the measuring conductor,
- wherein the signal paths for the reference signal and the measuring signal differ and the electrical signal path is extended by the measuring conductor,
- wherein a transit time of the run signal fed into the measuring conductor depends on the predetermined length of the measuring conductor and on a dielectric constant of the insulation of the measuring conductor, which varies with the ambient conditions, whereby a modified dielectric constant leads to a modified electrical path length.
- an evaluation unit for determining a measure of the modified condition based on a phase offset between the reference signal and the return signal obtained from the run signal, resulting from different propagation times due to the modified electrical path length.

10. Measuring arrangement according to claim 9, which comprises a summing or difference element for superimposing the return signal on the reference signal.

11. Measuring arrangement according to claim 9 or 10, wherein the measuring line is a stub line.

12. Measuring arrangement according to one of claims 9 to 11, in which the measuring line comprises two cores short-circuited to each other at the ends and, in particular, is designed as a two-wire line provided with a shielding.

13. Measuring arrangement according to one of claims 9 to 12, comprising a monitoring unit to which the measuring line is connected via contact connections, wherein in the monitoring unit the signal generator, the splitter, the receiver and the evaluation unit are integrated together.

14. Measuring arrangement according to one of claims 9 to 13, in which the measuring line is laid in a component to be monitored, in particular for monitoring a temperature prevailing in the component.

15. Measuring arrangement according to claim 14, in which the component is a cable, a thermally loaded component or a mass, which changes by an exothermic or endothermic reaction.

## Revendications

1. Procédé de surveillance d'une ligne pour des conditions ambiantes modifiées, dans lequel la ligne comprend une ligne de mesure avec une longueur prédéterminée qui comprend un conducteur de mesure entouré d'une isolation, dans lequel
- un signal analogique avec une fréquence déterminée est généré,
**caracterisé en ce que**
le signal est divisé en un signal de référence et un signal de marche, dans lequel les trajets de signal pour le signal de référence et le signal de mesure sont différents, dans lequel le trajet du signal électrique est prolongé par le conducteur de mesure,
- le signal de marche est introduit dans le conducteur de mesure,
- le signal introduit dans le conducteur de mesure passe par un trajet aller et un trajet retour et un signal de retour obtenu à partir du signal de marche et se propageant dans le trajet retour est combiné au signal de référence, dans lequel
- un temps de transit du signal de marche introduit dans le conducteur de mesure dépend de la longueur prédéterminée du conducteur de mesure et d'une constante diélectrique de l'isolation du conducteur de mesure, qui varie avec des conditions ambiantes, une constante diélectrique modifiée entraînant une longueur de trajet électrique modifiée
- sur la base d'un déphasage entre le signal de référence et le signal de retour résultant de différents temps de propagation dus à la modification de la longueur du trajet électrique, une mesure pour la condition modifiée est déterminée.

2. Procédé selon la revendication 1, dans lequel le signal de référence et le signal de retour sont superposés pour former un signal résultant et la mesure pour la condition modifiée est déterminée à partir de l'amplitude du signal résultant.

3. Procédé selon l'une des revendications 1 à 2, dans lequel le signal est un signal périodique avec une fréquence prédéterminée, notamment un signal sinusoïdal.

4. Procédé selon la revendication 3, dans lequel, dans des conditions normales, le signal de retour a un temps de transit prévu et la fréquence est réglée de telle sorte que, dans des conditions normales, une différence de phase définie entre le signal de retour et le signal de référence se produit, qui est de préférence de 90° ou 270°.

5. Procédé selon l'une des revendications 3 ou 4, dans lequel, dans des conditions normales, le signal de retour a un temps de transit prévu et, dans des conditions ambiantes s'écartant des conditions normales, une différence de temps de transit maximale prévue et dans lequel la fréquence est accordée à la différence de temps de transit maximale prévue de telle sorte que le déphasage possède une valeur de ≤180°, de préférence de ≤90° et notamment de ≤45°.

6. Procédé selon l'une des revendications 1 à 5, dans lequel le signal de retour est amplifié, notamment de sorte à avoir la même amplitude que le signal de référence.

7. Procédé selon l'une des revendications 1 à 6, dans lequel le signal de retour est une partie réfléchie du signal courant et la ligne de mesure est conçue comme un conduit de branchement.

8. Procédé selon l'une des revendications 1 à 7, dans lequel le signal de retour est couplé dans un conducteur de retour séparé du conducteur de mesure.

9. Dispositif de mesure pour la surveillance d'une ligne pour des conditions ambiantes modifiées, comprenant
- une ligne de mesure avec une longueur prédéterminée, ayant un conducteur de mesure entouré par une isolation,
- un générateur de signal pour générer un signal analogique avec une fréquence prédéterminée,
**caracterisé en ce que** le dispositif de mesure comprend en outre
- un séparateur pour générer un signal de référence et un signal de marche à partir du signal analogique,
- dans lequel le conducteur de mesure est connecté à une sortie du séparateur pour coupler le signal de marche dans le conducteur de mesure
- dans lequel les trajets du signal de référence et du signal de mesure diffèrent et le trajet du signal électrique est prolongé par le conducteur de mesure,
- dans lequel un temps de transit du signal de transit introduit dans le conducteur de mesure dépend de la longueur prédéterminée du conducteur de mesure et d'une constante diélectrique de l'isolation du conducteur de mesure, qui varie avec des conditions ambiantes, une constante diélectrique modifiée entraînant une longueur de trajet électrique modifiée.
- une unité d'évaluation pour déterminer une mesure pour la condition modifiée sur la base d'un décalage de phase entre le signal de référence et le signal de retour obtenu à partir du signal de marche, résultant de différents temps de propagation dus à la longueur modifiée du trajet électrique.

10. Dispositif de mesure selon la revendication 9, comprenant un élément de sommation ou de différence pour superposer le signal de retour au signal de référence.

11. Disposition de mesure selon la revendication 9 ou 10, dans lequel la ligne de mesure est un conduit de branchement.

12. Dispositif de mesure selon l'une des revendications 9 à 11, dans lequel la ligne de mesure comprend deux fils court-circuités l'un par rapport à l'autre aux extrémités et est conçue notamment comme une ligne bifilaire munie d'un blindage.

13. Dispositif de mesure selon l'une des revendications 9 à 12, comprenant une unité de surveillance à laquelle la ligne de mesure est reliée par des connexions de contact, le générateur de signal, le séparateur, le récepteur et l'unité d'évaluation étant intégrés ensemble dans l'unité de surveillance.

14. Dispositif de mesure selon l'une des revendications 9 à 13, dans lequel la ligne de mesure est posée dans un composant à surveiller, notamment pour surveiller une température régnant dans le composant.

15. Dispositif de mesure selon la revendication 14, dans lequel le composant est un câble, un composant chargé thermiquement ou une masse qui change par une réaction exothermique ou endothermique
